# EUROPÄISCHE PATENTSCHRIFT

(11) **EP 0 730 024 B1**
(45) Veröffentlichungstag und Bekanntmachung des Hinweises auf die Patenterteilung: **13.10.1999**
(21) Anmeldenummer: 95102920.6
(22) Anmeldetag: 01.03.1995
(51) Int. Cl.: C11D 3/386, C11D 3/00

(54) **Reinigungsmittel für chirurgische Instrumente**
Surgical instruments cleaning composition
Composition nettoyante pour instruments chirurgicaux

(43) Veröffentlichungstag der Anmeldung: 04.09.1996
(73) Patentinhaber: CHEMISCHE FABRIK DR. WEIGERT (GMBH & CO.), 20539 Hamburg (DE)
(72) Erfinder: Smitkowski, Petra, D-21033 Hamburg (DE); Schreiber, Olaf, D-22145 Hamburg (DE); Staffeldt, Jürgen, Dr., D-21423 Winsen/Luhe (DE)
(74) Vertreter: Glawe, Delfs, Moll & Partner

(56) Entgegenhaltungen:
- EP-A- 0 141 607
- EP-A- 0 199 404
- EP-A- 0 481 663
- WO-A-96/12000
- US-A- 4 243 546
- DATABASE WPI Week 9410, Derwent Publications Ltd., London, GB; AN 94-080216 & JP-A-6 033 098 (SHISEIDO CO LTD) 8. Februar 1994
- DATABASE WPI Week 9410, Derwent Publications Ltd., London, GB; AN 94-08209 & JP-A-6 033 090 (SHIS KK) 8. Februar 1994
- DATABASE WPI Week 9236, Derwent Publications Ltd., London, GB; AN 92-295468 & JP-A-4 202 300 (SHINETSU CHEM IND CO LTD) 23. Juli 1992

## Beschreibung

Die Erfindung betrifft ein Reinigungsmittelkonzentrat, insbesondere für die maschinelle Reinigung von medizinischen und/oder chirurgischen Instrumenten und/oder Apparaten sowie ein Verfahren zum Durchführen dieser Reinigung.

Chirurgische Instrumente sowie andere medizinische Geräte werden im Krankenhaus üblicherweise unter Verwendung alkalischer Reinigungsmittel maschinell gereinigt und anschließend chemisch oder thermisch desinfiziert. Häufig läßt sich mit solchen alkalischen Mitteln keine ausreichende Reinigungswirkung erzielen. Dies ist insbesondere dann der Fall, wenn mit Blut verunreinigte chirurgische Instrumente unmittelbar nach ihrer Benutzung bspw. in eine aldehydhaltige Desinfektionslösung abgelegt werden und darin zunächst verbleiben, bis sie zum Reinigen in die Spülmaschine geräumt werden. Das Blut wird durch die Desinfektionsmittel koaguliert und die im Blut enthaltenen Eiweißbestandteile durch den aldehydischen Desinfektionswirkstoff denaturiert. Solche besonders hartnäckigen Blutrückstände sind im Stand der Technik nur durch alkalischaktivchlorhaltige Reinigungsmittel zu entfernen. Die oxydierende Aktivchlorkomponente bewirkt die Zersetzung der denaturierten Einweißbestandteile.

Die alkalisch-aktivchlorhaltigen Reiniger weisen die Nachteile auf, daß sie kennzeichnungspflichtige Gefahrenstoffe enthalten, daß bei ihrer Handhabung besondere Vorsichtsmaßnahmen zum Schutz des Bedienungspersonals erforderlich sind und daß sie im Abwasser eine unerwünschte Umweltbelastung darstellen.

Aus offenkundiger Vorbenutzung sind ebenfalls enzymhaltige Reinigungsmittel bekannt. Die Reinigungswirkung solcher enzymatischer Mittel des Standes der Technik ist jedoch insbesondere für die Entfernung koagulierter und denaturierter Blutrückstände unzureichend.

WO-A-96 12000 (Stand der Technik gemäß Artikel 54 (3) EPÜ) offenbart Reinigerzusammensetzungen, die Amine und anionische oberflächenaktive Stoffe (z.B. Alkylsulfate mit mehr als 12 C-Atomen) enthalten. EP-A-0 481 663 offenbart ein zweistufiges Verfahren zum Reinigen chirurgischer Instrumente, bei dem diese zunächst in einer enzymatischen Lösung eingeweicht werden, dieser Lösung wird dann ein mit den Enzymen kompatibles Desinfektionsmittel zugegeben.

Der Erfindung liegt die Aufgabe zugrunde, ein Reinigungsmittelkonzentrat und ein Verfahren zum Reinigen von medizinischen und/oder chirurgischen Instrumenten und/oder Apparaten zu schaffen, bei dem die genannten Nachteile nicht oder in geringerem Maße auftreten und daß dennoch eine ausreichende Reinigungswirkung gewährleistet.

Die Erfindung löst diese Aufgabe durch die Merkmale der unabhängigen Ansprüche 1 und 7.

Gegenstand der Erfindung ist ein Reinigungsmittelkonzentrat mit folgender Zusammensetzung:
0,5-8,0 Gew.-% wenigstens eines C₅-C₁₂-Alkylsulfat-Salzes
4,0-15,0 Gew.-% wenigstens eines Lösungsvermittlers ,
4,0-10,0 Gew.-% wenigstens eines Alkanolamins, sowie
wenigstens ein handelsübliches proteolytisches Enzym in einer Menge von 0,005 - 0,1 Anson-Einheiten pro g Konzentrat
sowie gegebenenfalls
- übliche Säuren oder Basen zur pH-Wert-Einstellung,
- übliche Komplexbildner (bevorzugt in einer Menge von bis zu 10 Gew.-%) und
- übliche Konservierungsmittel,
- Rest, auf 100 Gew.-%, Wasser.

Überraschenderweise hat sich gezeigt, daß sich mit diesem nur schwach alkalischen Reinigungsmittel nicht nur an der Luft angetrocknete Blutrückstände, sondern auch vorerhitzte oder mit aldehydischen Desinfektionswirkstoffen denaturierte Blutrückstände von chirurgischen Instrumenten mittels einer maschinellen Reinigung einwandfrei entfernt werden können. Für den Erfolg der Erfindung ist die beanspruchte spezifische Kombination eines ionischen Tensids (des Alkylsulfat-Salzes), des Ethanolamins sowie des proteolytischen Enzyms wesentlich.

Vorzugsweise enthält das Reinigungskonzentrat 1 bis 6 Gew.-%, weiter vorzugsweise 2 bis 5 Gew.-%, C₅-C₁₂-Alkylsulfat-Salz. Bevorzugt werden Natrium, Kalium oder Ammoniumsalze verwendet. Die Kettenlänge der verwendeten Alkylsulfatsalze liegt vorteilhafterweise im Bereich C₅-C₁₀. Besonders bevorzugt werden Isooctylsulfate, Amylsulfate sowie Gemische daraus.

Geeignete Lösungsvermittler sind bspw. Natriumcumolsulfonat, Natriumtoluolsulfonat, Natriumxylolsulfonat, Harnstoff, Glykole, insbesondere Polypropylenglykole und Polyethylenglykole, Methylacetamid und Fettalkohole, wie bzw. Cetylalkohol. Ein bevorzugter Lösungsvermittler ist Natriumcumolsulfonat. Vorteilhafterweise sind die Lösungsvermittler in dem Reinigungsmittelkonzentrat in einer Menge von 6 bis 10 Gew.-% enthalten.

Das Alkanolamin (bevorzugt Mono-, Di- und/oder Triethanolamin) wird vorteilhaft in einer Menge von 6 bis 9 Gew.-% eingesetzt. Ein besonders bevorzugtes Alkanolamin ist das Triethanolamin.

Als proteolytische Enzyme sind insbesondere aus Bakterienstämmen gewonnene, handelsübliche Proteasen geeignet.

Bei dem ggf. zugesetzten Komplexbildnern kann es sich um Homo-, Co- oder Terpolymere auf der Basis von Acrylsäure oder deren Alkalisalzen handeln, ferner um Phosphonsäuren bzw. deren Alkalisalze, wie bspw. 1-Hydroxyethan-1,1-diphosphonsäure, Aminotrismethylenphosphonsäure, Ethylendiaminotetrakismethylenphosphonsäure, Phosphonobutantricarbonsäure; Weinsäure, Citronensäure und Glukonsäure; ferner Nitrilotriessigsäure oder Ethylendiaminotetraessigsäure bzw. deren Salze.

Als Konservierungsmittel sind bspw. p-Hydroxybenzoesäure oder deren Methylester, 5-Brom-5-Nitro-1,3-dioxan, Salicylsäure, 2-Naphtyl-m-N-Dimethylthiocarbanilat, 5-Chlor-5-methyl-4-isothiazolin-3-on, 2-Methyl-4-isothiazolin-3-on sowie Gemische der beiden letztgenannten Verbindungen. Ein bevorzugtes Konservierungsmittel ist p-Hydroxybenzoesäure bzw. deren Methylester. Mit Hilfe dieser Konservierungsmittel läßt sich Mikroben- und Pilzbefall des enzymhaltigen Reinigungsmittelkonzentrats vermeiden.

Die Erfindung betrifft ferner ein Verfahren zum insbesondere maschinellen Reinigen von medizinischen und/oder chirurgischen Instrumenten und/oder Apparaten, das durch folgende Schritte gekennzeichnet ist:
a) Aufbringen einer 0,5 - 100%igen wäßrigen Lösung eines Reinigungsmittelkonzentrats nach einem der Ansprüche 1 - 6, die einen pH-Wert von 7,5 - 10, bevorzugt von 8 - 9, aufweist,
b) Einwirkenlassen der Lösung bei einer Temperatur von Raumtemperatur bis 55°C für einen Zeitraum von 2 s - 20 min,
c) Nachspülen,
d) ggf. eine Thermodesinfektion.

Das Aufbringen der wäßrigen Lösung des Reinigungsmittelkonzentrats geschieht vorzugsweise durch Sprühen, kann aber bspw. auch durch Eintauchen oder Begießen erfolgen. Das Konzentrat kann in einer sehr hohen Konzentration, ggf. pur, als feiner Nebel aufgesprüht werden und zunächst in dieser hohen Konzentration oder pur einwirken. Anschließend kann ggf. zusätzliches Wasser auf die zu reinigenden Instrumente aufgebracht werden und die so verdünnte Reinigungslösung wird umgewälzt und bspw. durch Besprühen erneut auf die zu reinigenden Instrumente aufgebracht.

Der in Schritt b) definierte Einwirkzeitraum kann nach dem Aufbringen der Reinigungslösung ein Einwirken in Ruhe, d.h. ohne fortlaufendes Aufbringen bzw. Aufsprühen oder mechanisches Umwälzen bzw. Bewegen der Reinigungslösung umfassen. So kann bspw. insbesondere hochkonzentrierte Reinigungslösung zunächst aufgesprüht werden, nach dem Aufsprühen wird sie dann einwirken gelassen. Der Einwirkzeitraum kann aber auch Abschnitte umfassen, in denen die Reinigungslösung fortlaufend erneut auf die Instrumente aufgebracht bzw. aufgesprüht wird und/oder mechanisch umgewälzt oder auf irgendeine andere Art und Weise bewegt wird. Vorteilhaft ist auch eine Kombination dieser beiden Einwirkarten, also zunächst ein Einwirkenlassen insbesondere hochkonzentrierter Reinigerlösung in Ruhe und anschließendes Verdünnen des Reinigers mit Wasser unter laufender Umwälzung und erneutem Besprühen.

Die wäßrige Lösung des Reinigungsmittelkonzentrats wird vorteilhafterweise als 0,5 bis 20%ige, vorzugsweise als 0,5 bis 10%ige, weiter vorzugsweise als 1 bis 5%ige wäßrige Lösung aufgebracht. Während des Einwirkzeitraumes kann die Lösung des Konzentrats durch zusätzliches Wasser weiter verdünnt werden, dabei sollte aber eine Minimalkonzentration von 0,5% nicht unterschritten werden. Es sei noch angemerkt, daß alle Prozentangaben Gewichtsprozente sind.

Das Einwirkenlassen in Schritt b) geschieht vorzugsweise bei 35 - 50°C, weiter vorzugsweise bei 40 - 50°C. Einwirktemperaturen von etwas über 40°C haben sich als besonders vorteilhaft herausgestellt, da dabei einerseits eine gute Reinigungswirkung erzielt wird und andererseits die zu reinigenden Instrumente geschont werden.

Vorteilhafterweise beträgt die Einwirkzeit in Schritt b) 10 s bis 10 min, vorzugsweise 30 s bis 5 min.

Die Thermodesinfektion gemäß Schritt d) geschieht vorzugsweise mit vollentsalztem Wasser bei 85 - 95°C, vorzugsweise 93°C. Dieses Wasser kann gleichzeitig zum Nachspülen gemäß Schritt c) verwendet werden, so daß auf diese Weise die Schritte c) und d) kombiniert werden. Die Desinfektion kann auch auf andere Weise, bspw. durch chemische Desinfektionsmittel, erfolgen.

Im Rahmen der Erfindung kann die Reihenfolge der Schritte c) und d) auch vertauscht werden. Der Thermodesinfektionsschritt kann ggf. statt mit frischem Wasser auch mit der auf die entsprechende Temperatur (ca. 93°C) aufgeheizten Reinigungslösung erfolgen.

Ggf. können bei Verwendung sogenannter Taktbandspülmaschinen auch mehrere Reinigungsschritte mit dem erfindungsgemäßen Reinigungsmittelkonzentrat hintereinander ausgeführt werden. Dabei ist es bspw. möglich, in einem Reinigungsschritt ein Ultraschalltauchbad, das mit einer wäßrigen Lösung des erfindungsgemäßen Reinigungsmittelkonzentrats gefüllt ist, zu verwenden.

Die Erfindung erzielt eine gute Reinigungswirkung, insbesondere auch in schwer zugänglichen Bereichen chirurgischer Instrumente, bspw. im Gelenkbereich von Scheren. Insbesondere diese Bereiche lassen sich mit herkömmlichen Reinigern nur schwer reinigen.

Die Erfindung wird im folgenden anhand eines bevorzugten Ausführungsbeispiels näher erläutert.

Es wurde ein Reinigungsmittelkonzentrat der folgenden Zusammensetzung zubereitet:
Citronensäure 1,6 Gew.-%
NORAMER^{R 1)} 2000 (handelsüblicher Komplexbildner auf Basis von Carboxylat/Sulfonat-Acrylcopolymeren)
Sulfetal^{R 2)} 3,8 Gew.-%
Na-Cumolsulfonat ³⁾ 8,0 Gew.-% p-Hydroxybenzoesäure 0,5 Gew.-%
Triethanolamin 7,5 Gew.-%
Esperase 8.0 L⁴⁾ 2,0 Gew.-%
Rest (auf 100 Gew.-%) Wasser
¹⁾ Firma NorsoHaas S.A., Verneuil, En Halatte, Frankreich
²⁾ Gemisch aus Isooctylsulfat-Na- und Amylsulfat-Na-Salz, 38%ig, berechnet auf 100%ige Konzentration
³⁾ 40%ig, berechnet als 100%iges Präparat
⁴⁾ Firma Novo Industries, Dänemark

Das bei diesem Ausführungsbeispiel verwendete Wasser ist Hamburger Stadtwasser.

### Vergleichsversuche zur Reinigungswirkung

Edelstahlplättchen werden mit einer Prüfanschmutzung von 0,1 ml frischem Schweineblut pro Plättchen versehen. Das Schweineblut wird bei Raumtemperatur 40 min lang angetrocknet. Die Prüfplättchen werden anschließend 1 min lang in einer 0,1%igen Lösung von Glutardialdehyd getaucht und bei Raumtemperatur weitere 60 min getrocknet. Die Glutardialdehydlösung denaturiert den Eiweißgehalt des Blutes und stellt so die Bedingungen ein, die auch bei in Desinfektionsmitteln aufbewahrten benutzten chirurgischen Instrumenten vorliegen.

Es werden vier wäßrige Reinigungslösungen zubereitet:
1. eine 10%ige Lösung des erfindungsgemäßen Konzentrats gemäß dem Ausführungsbeispiel
2. eine 10%ige Lösung eines handelsüblichen alkalischen Reinigers folgender Zusammensetzung:
   46% Pentakaliumtriphosphat (50%ig)
   24% Kalilauge (45%ig)
   30% Natriumwasserglas (38°Be')
3. eine 10%ige Lösung eines handelsüblichen alkalischen Intensivreinigers der Zusammensetzung:
   40% Nitilotriessigsäure, Na-Salz (40%ig)
   32% Natronlauge (45%ig)
   4% amphoteres Tensid (40%ig)
   Rest (auf 100%) Wasser
4. eine 10%ige Lösung eines handelsüblichen alkalischen aktivchlorhaltigen Reinigers mit der Zusammensetzung:
   16% Kalilauge (45%ig)
   28% Pentakaliumtriphosphat (50%ig)
   16% Wasserglas (38°Be')
   5% Pentanatriumtriphosphat
   21% Chlorbleichlauge (150 - 160 g Aktivchlor/l)
   Rest (auf 100%) Wasser

Die Lösungen werden auf 40°C erwärmt und jeweils zwei angeschmutzte Prüfplättchen in die ruhende Lösung eingetaucht. Die Plättchen werden nach 5 bzw. 10 min Einwirkzeit wieder herausgenommen.

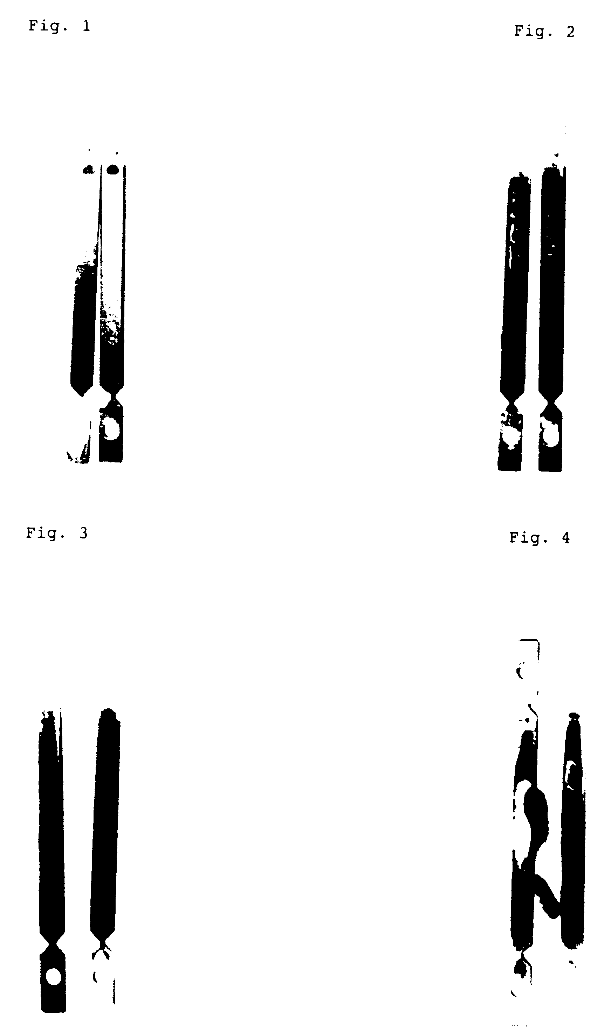

Die Reinigungswirkungen der Lösungen 1 bis 4 sind in den Fig. 1 bis 4 dargestellt. In jeder der Figuren weist das linke Prüfplättchen eine Einwirkzeit von 5 min, das rechte eine Einwirkzeit von 10 min auf.

Man erkennt deutlich, daß die alkalischen Reiniger nur eine schlechte Reinigungswirkung zeigen und noch dicke Krusten vorhanden sind, die allenfalls abblättern. Nach der Behandlung mit dem erfindungsgemäßen Reinigungskonzentrat sind nur noch geringe Restspuren von denaturiertem Blut vorhanden, der größte Teil des Blutes ist abgelöst worden. Bei der Beurteilung dieser Vergleichsversuche ist zu berücksichtigen, daß sich in der Praxis beim maschinellen Reinigen aufgrund der Bewegung und Umwälzung der Reinigungsmittellösung deutlich bessere Reinigungswirkungen ergeben.

### Verfahrensbeispiele für maschinelles Reinigen

1. In einer Eintank-Spülmaschine werden die zu reinigenden Instrumente zunächst mit Kaltwasser vorgespült. Anschließend wird die Spülmaschine mit kaltem Wasser gefüllt und das Reinigungsmittelkonzentrat gemäß dem Ausführungsbeispiel in einer Konzentration von 1,5% zudosiert. Die Reinigungslösung wird auf 40 - 45°C aufgeheizt und 5 min bei dieser Temperatur gehalten. Anschließend wird mit Wasser nachgespült. Zum Schluß erfolgt eine Thermodesinfektion mit vollentsalztem Wasser bei 93°C. Mit diesem Wasser wird gleichzeitig nachgespült.
2. Reinigung unter Verwendung einer Taktbandanlage
   Bei einer Taktbandanlage werden die zu reinigenden Instrumente nacheinander in verschiedene Reinigungskammern gefahren. Bei diesem Verfahrensbeispiel wird eine 3-KammerAnlage eingesetzt.
   In der ersten Kammer wird zunächst eine Vorreinigung mit kaltem Wasser für einem Zeitraum von 30 s vorgenommen, anschließend erfolgt eine Reinigung mit einer 1%igen wäßrigen Konzentration des Reinigungsmittelkonzentrats gemäß dem Ausführungsbeispiel für einen Zeitraum von 5 min bei einer Temperatur von 40°C. Im Anschluß daran wird wieder Wasser 30 s lang nachgespült.
   In der zweiten Kammer werden die Instrumente mit einer 2%igen wäßrigen Lösung des Reinigungsmittelkonzentrats gemäß dem Ausführungsbeispiel bei 35°C 5,5 min in einem Ultraschalltauchbad behandelt.
   In der dritten Kammer erfolgt eine 6-minütige Nachspülung und gleichzeitige Thermodesinfektion mit vollentsalztem Wasser bei 93°C.

## Patentansprüche

1. Reinigungsmittelkonzentrat mit folgender Zusammensetzung:
0,5-8,0 Gew.-% wenigstens eines C₅-C₁₂-Alkylsulfat-Salzes,
4,0-15,0 Gew.-% wenigstens eines Lösungsvermittlers,
4,0-10,0 Gew.-% wenigstens eines Alkanolamins, sowie
wenigstens ein handelsübliches proteolytisches Enzym in einer Menge von 0,005 - 0,1 Anson-Einheiten pro g Konzentrat
sowie gegebenenfalls
- übliche Säuren oder Basen zur pH-Wert-Einstellung,
- übliche Komplexbildner und
- übliche Konservierungsmittel,
- Rest, auf 100 Gew.-%, Wasser,
wobei das Konzentrat keine Alkylsulfate mit mehr als 12 C-Atomen enthält.

2. Reinigungsmittelkonzentrat nach Anspruch 1, gekennzeichnet durch einen Gehalt von 1 - 6 Gew.-%, vorzugsweise 2 - 5 Gew.-%, C₅-C₁₂ Alkylsulfat-Salz.

3. Reinigungsmittelkonzentrat nach Anspruch 1 oder 2, gekennzeichnet durch einen Gehalt von 6 - 10 Gew.-% Lösungsvermittler.

4. Reinigungsmittelkonzentrat nach Anspruch 1 bis 3, dadurch gekennzeichnet, daß als Lösungsvermittler Natriumcumolsulfonat verwendet wird.

5. Reinigungsmittelkonzentrat nach Anspruch 1 bis 4, gekennzeichnet durch einen Gehalt von 6 - 9 Gew.-% Alkanolamin.

6. Reinigungsmittelkonzentrat nach Anspruch 1 - 5, dadurch gekennzeichnet, daß das Konzentrat Mono-, Di- und/oder Triethanolamin enthält.

7. Verfahren zum Reinigen von medizinischen und/oder chirurgischen Instrumenten und/oder Apparaten, gekennzeichnet durch folgende Schritte:
a) Aufbringen einer 0,5 - 100%igen wäßrigen Lösung eines Reinigungsmittelkonzentrats nach einem der Ansprüche 1 - 6, die einen pH-Wert von 7,5 - 10, bevorzugt von 8 - 9, aufweist,
b) Einwirkenlassen der Lösung bei einer Temperatur von Raumtemperatur bis 55°C für einen Zeitraum von 2 s - 20 min,
c) Nachspülen,
d) ggf. eine Thermodesinfektion.

8. Verfahren nach Anspruch 7, dadurch gekennzeichnet, daß das Reinigungsmittelkonzentrat in Schritt a) als 0,5 - 20%ige, vorzugsweise 0,5 - 10%ige, weiter vorzugsweise 1 - 5%ige wäßrige Lösung aufgebracht wird.

9. Verfahren nach Anspruch 7 oder 8, dadurch gekennzeichnet, daß das Einwirkenlassen in Schritt b) bei 35 - 50°C, vorzugsweise bei 40 - 50°C geschieht.

10. Verfahren nach Anspruch 7 bis 9, dadurch gekennzeichnet, daß die Einwirkzeit in Schritt b) 10 s - 10 min, vorzugsweise 30 s - 5 min beträgt.

## Claims

1. A cleansing agent concentrate having the following composition:
0.5-8.0 % by weight of at least one C₅-C₁₂ alkyl sulphate salt,
4.0-15.0 % by weight of at least one solubiliser,
4.0-10.0 % by weight of at least one alkanol amine, and also
at least one commercially available proteolytic enzyme in a quantity of 0.005 to 0.1 Anson units per g of concentrate
as well as optionally
- conventional acids or bases for pH value adjustment,
- conventional complexing agents and
- conventional preserving agents,
- the remainder water, up to 100 % by weight,
wherein the concentrate does not contain any alkyl sulphates with more than 12 C atoms.

2. A cleansing agent concentrate according to Claim 1, characterised by a content of 1 to 6 % by weight, preferably 2 to 5 % by weight, C₅-C₁₂ alkyl sulphate salt.

3. A cleansing agent concentrate according to Claim 1 or 2, characterised by a content of 6 to 10 % by weight of solubiliser.

4. A cleansing agent concentrate according to Claims 1 to 3, characterised in that sodium cumene sulphonate is used as solubiliser.

5. A cleansing agent concentrate according to Claims 1 to 4, characterised by a content of 6 to 9 % by weight of alkanol amine.

6. A cleansing agent concentrate according to Claims 1 to 5, characterised in that the concentrate contains mono, di and/or triethanol amine.

7. A method of cleansing medical and/or surgical instruments and/or apparatus, characterised by the following steps:
a) applying a 0.5 to 100 % aqueous solution of a cleansing agent concentrate according to any one of Claims 1 to 6, which has a pH value of 7.5 to 10, preferably 8 to 9,
b) allowing the solution to act at a temperature of from room temperature to 55°C for a period of 2 s to 20 min.,
c) rerinsing,
d) optionally a thermal disinfection.

8. A method according to Claim 7, characterised in that the cleansing agent concentrate in step a) is applied as a 0.5 to 20 %, preferably 0.5 to 10 %, more preferably 1 to 5 % aqueous solution.

9. A method according to Claim 7 or 8, characterised in that the action in step b) takes place at 35 to 50°C, preferably at 40 to 50°C.

10. A method according to Claims 7 to 9, characterised in that the acting time in step b) is 10 s to 10 min., preferably 30 s to 5 min.

## Revendications

1. Concentré de composition nettoyante, ayant la composition suivante :
0,5-8,0% en poids d'au moins un sel d'alcoylsulfate en C₅-C₁₂ ;
4,0-15,0% en poids d'au moins un agent de solubilisation ;
4,0-10,0% en poids d'au moins une alcanolamine, ainsi que
au moins une enzyme protéolytique commerciale en une quantité allant de 0,005 à 0,1 unité Anson par g de concentré, ainsi que facultativement,
des acides ou bases usuelles pour l'ajustage du pH ;
un agent complexant usuel, et
un agent de conservation usuel,
le reste, jusqu'à 100% en poids, étant de l'eau,
où le concentré ne contient pas d'alcoylsulfate ayant plus de 12 atomes C.

2. Concentré de composition nettoyante suivant la revendication 1, caractérisé par une teneur allant de 1 à 6% en poids, de préférence de 2 à 5% en poids, en sel d'alcoylsulfate en C₅-C₁₂.

3. Concentré de composition nettoyante suivant la revendication 1 ou 2, caractérisé par une teneur allant de 6 à 10% en poids en agent de solubilisation.

4. Concentré de composition nettoyante suivant les revendications 1 à 3, caractérisé en ce que l'on utilise du cumènesulfonate de sodium comme agent de solubilisation.

5. Concentré de composition nettoyante suivant les revendications 1 à 4, caractérisé par une teneur allant de 6 à 9% en poids en alcanolamine.

6. Concentré de composition nettoyante suivant les revendications 1 à 5, caractérisé en ce que le concentré contient de la mono-, di- et/ou triéthanolamine.

7. Procédé de nettoyage des instruments et/ou appareils médicaux et/ou chirurgicaux, caractérisé par les étapes consistant à :
a) disposer une solution aqueuse à 0,5-100% d'un concentré de composition nettoyante suivant l'une quelconque des revendications 1 à 6, laquelle présente un pH allant de 7,5 à 10, de préférence de 8 à 9 ;
b) laisser agir la solution à une température allant de la température ambiante à 55°C pendant une période allant de 2 secondes à 20 minutes,
c) rincer ;
d) facultativement, désinfecter de manière thermique.

8. Procédé suivant la revendication 7, caractérisé en ce que le concentré de composition nettoyante est disposé à l'étape a), sous forme d'une solution aqueuse à 0,5-20%, de préférence 0,5-10%, de manière davantage préférée 1-5%.

9. Procédé suivant la revendication 7 ou 8, caractérisé en ce que la période d'action de l'étape b) se produit à 35-50°C, de préférence à 40-50°C.

10. Procédé suivant les revendications 7 à 9, caractérisé en ce que la période d'action de l'étape b) se situe dans l'intervalle allant de 10 secondes à 10 minutes, de préférence de 30 secondes à 5 minutes.
